# EUROPEAN PATENT SPECIFICATION

(11) **EP 1 230 392 B1**
(45) Date of publication and mention of the grant of the patent: **07.07.2010**
(21) Application number: 00977665.9
(22) Date of filing: 20.11.2000
(51) Int. Cl.: C12Q 1/68

(54) **SECURITY SYSTEM**
SICHERHEITSSYSTEM
SYSTEME DE SECURITE

(30) Priority: 19.11.1999 GB 9927292
(43) Date of publication of application: 14.08.2002
(73) Proprietor: Crime Solutions Limited, Coventry CV5 9EN (GB)
(72) Inventor: BROWN, Tom, Southampton SO16 7JD (GB); THELWELL, Nichola, Glossop Derbyshire SK13 7QU (GB); MAXWELL, Paul, Coventry CV5 9EN (GB); WHITING, Paul, Coventry CV3 4HD (GB); MAXWELL, Paula, Coventry, CV5 6GE (GB)
(74) Representative: MacLean, Martin Robert
(86) International application number: PCT/GB2000/004419
(87) International publication number: WO 2001/036676

(56) References cited:
- WO-A-95/02702
- US-A- 5 451 505
- US-A- 5 643 728
- DATABASE EMBL [Online] EMBL; Database entry/accession number AB011218, 7 August 1998 (1998-08-07) "Bradypus variegatus mitochondrial ND1 gene for NADH dehydrogenase subunit 1" XP002169844 & CAO YING ET AL: "Conflict among individual mitochondrial proteins in resolving the phylogeny of eutherian orders." JOURNAL OF MOLECULAR EVOLUTION, vol. 47, no. 3, 1998, pages 307-322, ISSN: 0022-2844

## Description

The present invention relates to security markers for marking objects and people, methods of using the markers, methods of detecting the markers and security devices containing such markers. In particular, the security markers used comprise a nucleic acid molecule which can be uniquely identified to a specific source of the security marker.

Security markers for identifying the ownership of an article are known in the art. Some markers use a specific formulation of different chemical compounds to identify that an object is owned by somebody or was at a specific location. Such chemical markers are shown, for example, in GB 2245583. Such systems have only a limited number of identifying compounds, thus making it difficult to uniquely identify a large number of locations.

Security markers comprising the use of nucleic acid molecules are also known in the art, as shown in, for example, WO 94/04918, WO 87/06383 and WO 95/02702. Such security markers may be simply identified by the use of a suitable probe which may hybridise to the nucleic acid within the marker. The use of polymerase chain reaction (PCR) to isolate and multiply a sample of nucleic acid within a security marker is known, as shown in WO 90/14441.

WO 95/02702 describes a method for marking articles with beads having nucleic acids attached thereto.

US 5,451,505 describes a method for tagging and tracing materials using nucleic acids as taggants.

US 5,643,728 describes a method for marking a liquid with a plurality of nucleic acid containing particles.

An advantage of using a nucleic acid molecule as a security marker is that a unique nucleotide sequence can be used for each user of the security marker or for each location in which a security marker is used. Use of oligonucleotides having random nucleic acid sequences is disclosed in US 5,139,812.

The inventors have realized that there is a market for improved security markers. Such security markers may be used to label goods or alternatively be used in a security system, whereby they are sprayed upon an intruder upon activation of a suitable actuator. In the latter case, such security markers are useful to demonstrate that an intruder was at the scene of a crime.

Where such a marker is used for the purposes of crime detection it is important that the detection of the marker is as error-free as possible to reduce the possibility of falsely convicting third parties.

The inventors have realised that polymerase chain reaction of a strand of nucleic acid using a suitable primer, followed by sequencing from that primer, does not always produce a consistent sequence for any marker DNA. It is therefore useful to have a backup to confirm the sequence of any unique sequence of a marker region of the security marker. The inventors have therefore provided a second primer region on the security marker which enables the sequence of the marker region to be confirmed by allowing the complementary strand to be produced by PCR and then to be sequenced using a second primer.

Accordingly, a first aspect of the invention provides method of producing a security marker for marking objects and/or people as set forth in the claims.

The terms "substantially identical to the first primer" or "substantially the reverse complement of a second primer" are intended to mean that there is sufficient homology to allow the first primer or second primer, and the region to which they bind on the security marker or a nucleic acid molecule complementary to the security marker, to hybridise under high stringency conditions. Such conditions are preferably under typical PCR conditions.

Preferably the security marker is a single stranded nucleic acid molecule which is easier to manufacture as oligonucleotide. Alternatively, double stranded markers may be used.

Preferably, in use, the second primer is used to allow the nucleic acid molecule to replicate by PCR, thus producing a second complementary strand of nucleic acid. The complementary strand then comprises a primer region which has the correct sequence to enable the first primer to bind with it. The first primer can then be used to multiply the complementary strand of nucleic acid.

Preferably the complementary primer region is downstream of the marker region.

The inventors have also realised that where a marker has been attached to an object or an intruder, for example, there is often a large amount of contaminating DNA fragments from, for example, human beings and/or any pets, such as dogs or cat, that are around the object or intruder.

Accordingly, the nucleic acid sequence of the first primer and the nucleic acid sequence of the second primer are selected so that there is a low probability that their respective primer binding regions occur with 150 bases or 120 or 100 bases of each other in native DNA. That is, naturally occurring sequences to which primers used with the security marker can bind under high stringency conditions do not normally occur within 150 bases of each other in native DNA.

Preferably the probability is less than 95%, especially less than 97%, more preferably less than 99%.

By high stringency we mean, for example, 1.5 mM MgCl₂, 53°C annealing temperature.

This has been achieved by carefully selecting the primers used against publicly available databases of nucleotide sequences. Preferably the native DNA is prokaryotic or eukaryotic, especially humans, dog, cat, mouse, rat and/or insect DNA.

This has the advantage that it is possible to rapidly identify amplified DNA fragments containing the marker region by size. One or other primer may bind to non-security marker DNA but to produce a PCR product requires both primers to bind but these are likely to only produce DNA of a different size to the security marker DNA, thus enabling such fragments to be easily discounted.

Once a sample containing a security marker has been identified, the security marker may then be sequenced to determine the exact sequence of the marker region.

Preferably the primers and the regions to which they bind on the security marker, or a complementary molecule, are 100% complementary to their binding region. This allows high levels of stringency to be used to assay the presence of marker.

In an especially preferred embodiment, the inventors have identified that nucleic acid from the brown throated sloth (*Bradypus variegatus*) The especially preferred sequence from the brown throated sloth used is a nucleic acid sequence corresponding to the mitochondrial ND1 gene of the sloth.

By nucleic acid we mean single or double stranded DNA or RNA of natural or synthetic origin, or synthetic derivatives thereof which may be totally or partially constituted of rare bases or by modified bases.

The primer regions are arranged on the nucleic acid to enable a suitable nucleic acid primer to selectively hybridise to the primer region, thus allowing the security marker containing the marker region to be amplified from a sample.

Preferably, the primer region consists of between 15 and 50, preferably 17-30, especially 18 nucleotides to allow sufficiently high stringency of hybridisation with a suitable primer used to identify the presence of the security marker. The primer itself may be used to directly amplify the security marker by, for example, polymerase chain reaction.

The first primer and second primer may be the same or different.

The marker region itself contains a predetermined nucleic acid sequence which is preferably unique to the owner of a particular security marker or to a particular location, such as a specific factory building or specific car. For example, where the security marker is used on an article and the article has been stolen and then recovered by police, after the security marker is identified by sequencing the marker sequence, the owner of the stolen article may be traced. Alternatively, for example, where the security marker has been sprayed onto an intruder, then the unique sequence of the security marker can identify that the intruder has been within a particular factory premises or within a car when, for example, a security device containing the security marker has been activated.

The term "complementary" defines the relationship between two strands of DNA or DNA and RNA where they are complementary to each other if their sequences are related by base-pairing rules. Thus, ATCG is complementary to the sequence TAGC and paired with it by hydrogen bonding. Accordingly, the accuracy of the sequencing of the marker region may be determined by comparing the sequence with the sequence obtained for the marker from the second primer and seeing whether the two sequences are complementary to each other.

Preferably, the marker region consists of between 10 and 30 nucleotides, especially between 15 and 25, more preferably 20 nucleotides in length. This allows a large number of unique sequences, each of which are unique to a particular application of the security marker to be generated.

The or each primer may be separated from the marker region by a stretch of up to 50, especially between 20 and 40, more preferably a stretch of 20 to 30 nucleotides in length. The stretch of nucleotides between the primer and the marker region have been found by the inventors to improve the accuracy of the sequencing of the security marker.

The nucleic acid molecule within the security marker may be labelled. It may, for example, be labelled with a visible dye which enables the security marker to be easily seen with the naked eye. Alternatively, the label may be a fluorescent dye which enables the nucleic acid molecule to be identified under ultraviolet light.

An example of suitable security markers are shown in table 1.

**Table 1**

| **Spectral characteristics of fluorescent dyes suitable for labelling oligonucleotides** | | | | |
|---|---|---|---|---|
| **Dye** | **Absorption wavelength** | **Emission wavelength** | **extinction coeff M⁻¹cm⁻¹** | **Quantum yield** |
| 5-or 6-FAM (fluorescein) | 500 | 525 | | |
| TET | 525 | 550 | | |
| JOE | 530 | 555 | | |
| HEX | 540 | 565 | | |
| TAMRA | 555 | 580 | | |
| ROX | 585 | 610 | | |
| BODIPY FL C3 | 502 | 510 | 82,400 | |
| BODIPY FL-X | 503 | 510 | 85,306 | |
| BODIPY TMR-X C3 | 544 | 570 | 55,586 | |
| BODIPY 493/503 | 493 | 503 | 79,169 | |
| BODIPY 530/550 | 530 | 550 | 77,000 | |
| BODIPY 558/568 | 558 | 568 | 95,700 | |
| BODIPY 564/570 | 564 | 570 | 141,650 | |
| BODIPY 576/589 | 576 | 589 | 83,400 | |
| BODIPY 581/591 | 581 | 591 | 135,900 | |
| BODIPY TR-X C3 | 587 | 616 | 67,700 | |
| Cy2 | 489 | 506 | 150,000 | >0.12 |
| Cy3 | 550 | 570 | 150,000 | >0.15 |
| Cy3.5 | 581 | 596 | 150,000 | >0.15 |
| Cy5 | 649 | 670 | 250,000 | >0.28 |
| Cy5.5 | 675 | 694 | 250,000 | >0.28 |
| Cy7 | 743 | 767 | 250,000 | 0.28 |
| FluorX | 494 | 520 | 68,000 | 0.3 |
| R6G (6-carboxy-rhodamine 6G | 535 | 555 | | |
| Texas red | 584 | 603 | 116,300 | |

Alternatively, the security marker may comprise one or more separate labels such as colorimetric and/or fluorescent labels.

The nucleic acid molecules, when exposed to the surface of any object, may be degraded by naturally occurring nucleases, such as 3'- or 5'-exonucleases. Such nucleases may be present on the skin of a person or from bacteria on the surface of an object. It is therefore desirable to improve the resistance of the security markers to attack from such enzymes. Accordingly, preferably the nucleic acid molecule comprises at one or both ends a blocking agent. Such blocking agents include those agents which are known to resist nuclease attack such as phosphothioate linkage, non-natural nucleosides, dendrimers or amino alkyl groups.

It is also desirable to make the security marker stick to an object onto which it has been attached. The nucleic acid molecule preferably comprises one or more binding agents which assists in attaching the nucleic acid molecule to an object. The binding agents may include biotin, vitamin-E, cholesterol and phosphothioates. Biotin, vitamin-E and cholesterol may be used since they are fat-soluble molecules which enable the nucleic acid molecule to bind to the skin of an intruder. Furthermore, biotin is especially useful because nucleic acid molecules containing biotin which have been obtained from a sample, may be readily isolated by running a solution made from the sample through an avidin-containing column. The biotin-containing nucleic acid molecule may bind to the avidin on the column, allowing it to be isolated and purified.

Phosphothioates are useful as binding-agents because the phosphothioate will form disulphide bonds with naturally occurring cysteine residues in proteins such as wool, hair, fingernails, etc.

The security marker may be removed from the surface of an object by any suitable means. For example, swabbing using ethanol has been found to work. Skin scrapes may also be used. Dithiothreitol (DTT) has been found to be useful in isolating security markers comprising phosphothioate incorporated into the nucleic acid molecule. The DTT denatures any disulphide bonds between the phosphothioate and proteins on the object which has been marked, thus releasing the security marker.

Different coloured or different fluorescently labelled security markers may be used for different situations. For example, a specific colour may be used in one country and not another, thus allowing the identification of crime suspects when they pass through, for example, customs. Alternatively, different colours may be used for different situations, for example to demonstrate whether a person has carried out a car theft or has been involved in breaking and entering a factory premises.

The invention further provides the use of a security marker according to the invention to mark an object.

A method of marking an object comprising applying a security marker, according to the invention, to an object is also provided. A security marker may be applied by any suitable means such as using a brush, a spray, an aerosol or a stream. Preferably, the object is a human, such as an intruder.

Methods of detecting security markers according to the invention also provided, the method comprising the steps of:
(i) obtaining a sample of an object which potentially has been labelled with a security marker according to the invention;
(ii) amplifying the marker with two primers capable of selectively binding to the marker nucleic acid; and
(iii) sequencing any security marker identified from at least one primer to determine the nucleic acid sequence of the marker region within the security marker.

The security marker may be amplified by, for example, PCR prior to sequencing the first marker region.

The security marker may additionally be screened with a second probe capable of hybridising to the second primer region once the marker has been replicated to form a complementary strand by e.g. PCR, and the second marker region is then sequenced.

The marker regions may be sequenced by any methods known in the art, such as dideoxy sequencing, mass-spectrometry sequencing or other techniques known in the art.

Such sequencing may occur after initial amplification by PCR or directly, e.g. using minisequencing.

The sequence of marker region may be compared with a database to determine the source of the security marker. That is, to enable the owner of the object to be identified, for example, or to identify where a prime suspect may have been exposed to the security marker.

A further aspect of the invention provides a security kit comprising a security marker according to the invention. The security kit may comprise a security marker within a suitable solution or, for example, within a grease. The grease may be of use to ensure that a sample of cloth is successfully marked.

The invention will now be described by way of example only with reference to the following figures.
**Figure 1** shows a schematic diagram representing a security marker according to the invention.
**Figure 2** shows (a) the nucleotide sequence obtained from a security marker according to the invention, and (b) the sequencing trace obtained.
**Figure 3** shows a flow diagram indicating the use of a security marker of the invention.
**Figure 4** shows a summary of the use of a security marker according to the invention.

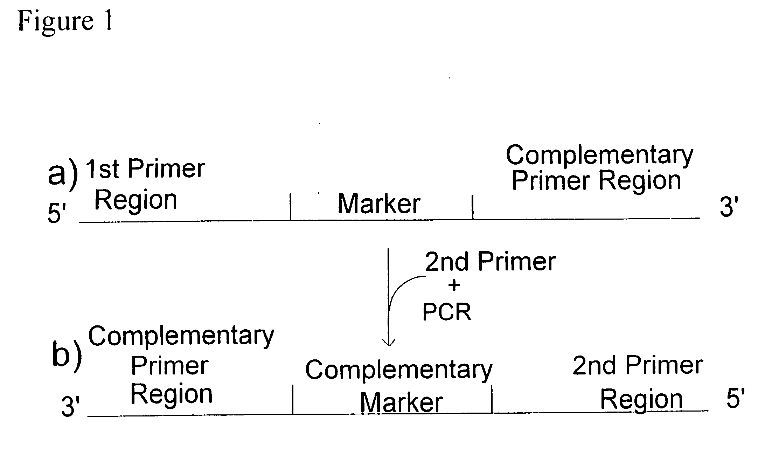

Figure 1a shows a single-stranded nucleic acid molecule. The nucleic acid molecule may be of DNA or RNA. The strand comprises a first primer region and, downstream of the primer region, a marker region and a complementary primer region. Upon carrying out PCR with a primer capable of binding the second primer region, a complementary strand (1b) is produced. The complementary strand has a functional first primer region capable of being bound by a first primer. It also has a marker region complementary to the marker region on the original marker.

The inventors have found that a particularly useful primer sequence may be obtained from the brown-throated sloth. This has the following sequence:

5'-ta cccacgattccgctacgatc aactaataca cttgctatga aaaaatttcc tcccactcac
cctggccctatgca cgtgacacataacc-3'

This is obtained from the mitochondrial ND1 gene of the organism.

This was used to create the following primers:
1. 5'-ta cccacgattccgctac-3'.
2. 5'-ggttatgtgtcacgtgca-3'.

The primer sequences were attached to unique marker sequences, for example of sequence:
gaaaaaatttcctcccactcac
which were separated from the primer sequences by a stretch of 22 bases from one primer region, 10 bases from a second primer region.

The nucleic acid molecules can be prepared by conventional solid-phase methods, typically on an automated DNA synthesiser. The most common method involves the use of β-cyanoethyl phosphoramidite chemistry as described in the following publications:
The synthesis of chemically labelled PCR primers. D.J.S. Brown and T. Brown (1995 "PCR: essential data" Wiley, ed. C.R. Newton. Chapter 7, pp 57-70.
Preparation of synthetic oligodeoxynucleotide probes. T. Brown and J. Grzybowski (1995) "Gene probes: a Practical Approach". IRL press. chapter 5, 146-167.

Oligonucleotides can also be synthesised by manual solid-phase methods or by solution-phase methods but the automated solid-phase method has the advantage of high throughput.

DNA isolated from biological sources can also be used.

The nucleic acid molecules optionally contained vitamin-E, cholesterol, phosphothioate and/or FAM (a fluorescein dye).

Samples of the security marker were placed upon samples such as fabric or skin.

### Polymerase Chain Reaction Protocol

Conventional PCR techniques were used to identify and multiply the sequences. Typical 50 µL reaction mix comprise.
10x PCR buffer developed for SYBR Green reactions on the ABI Prism Sequence detection system 7700, which was obtained from Genesys.
200 µM dNTP's (using a mixture of dUTP and dTTP)
0.5 µM concentration of both primers.
1.5 mM MgCl₂.
1 unit of Hot GoldStar enzyme (a TAQ polymerase)
0.5 units of UNG.

Typical cycling conditions were 53°C for 2 mins., 95°C for 10 mins., 40 cycles of 95° for 30 secs., 50° for 30 secs. and then 72°C for 30 secs. A final extension was carried out at 72°C for 7 mins.

The results were analysed for size of product using 5 µl of product on a 2% agarose gel.

Retrieval of a DNA from cotton fabric (5 µl of a solution of 0.04 ng/ml of DNA added to a small spot, allowed to dry and left on the lab bench) by soaking fabric in 1 ml of sterile water or ethanol for 1 hour, optionally followed by removal of fabric, is possible for at least 1 week. Where ethanol was used, this was followed by ethanol precipitation. The water was frozen until the end of the week when the PCR reactions were carried out together, using 5 µl of the water. All samples gave a good PCR product of the expected size.

Washing the fabric at 40°C reduced the amount of product, as does ironing and washing followed by ironing.

Biotin and Vitamin-E were found not to inhibit PCR or sequencing when attached to the 5' end of the target. The PCR products were sequenced following PCR purification using a Quiagen kit. Both gave sequences with the lower primer giving better sequence than the upper primer.

Biotin and Vitamin-E were found to remain on fabric (5 µl of 0.04 ng/µl) over 3 days and can be retrieved by soaking in 2 ml of water for 1 hr. followed by freezing of sample.

Biotin and Vitamin E targets could also be retrieved from skin by swabbing with damp cotton wool (non-absorbent) after 5 µl of 0.04 ng/µl were left to dry for 10 mins. on the surface of skin. Biotin gave the best result. There was no contamination when a patch of skin that had not been treated with target was swabbed.

After 24 hours (skin was treated normally, i.e. washed) biotin could be retrieved and gave a good PCR product, although less than after 10 mins.

Cholesterol could also be successfully retrieved from fabric and skin both immediately after application and after 24 hours using water or ethanol. It was also shown to remain on fabric after being washed at 60°C.

Two different markers were made which had been labelled with FAM. One was labelled with FAM, the other had FAM at one end and 3 phosphothioate bonds at the other end, between additional thymine bases added to the molecule. The phosphothioate bonds are thought to bind to proteins in the skin, making the security marker attach to it. FAM was attached using standard phosphoramidite chemistry with extended coupling time. Phosphothioate was added using a standard phosphothioate cycle, replacing iodine with TETD (tetraethyl thiuramdisulphide) as the oxidising agent.

The target with the phosphothioate bonds was successfully retrieved by swabbing skin with 1% DTT, followed by soaking the swab in 3 ml, of 70% ethanol and 0.3 M NaOAc.

Retrieval from fabric was undertaken by soaking the fabric in 2 ml. of 1% DTT for 10 mins.

The DNA from the sample was then retrieved by ethanol precipitation, using techniques known in the art.

Neither FAM or phosphothioate bonds were observed to inhibit PCR.

Furthermore, PCR products were successfully retrieved using the system described above.

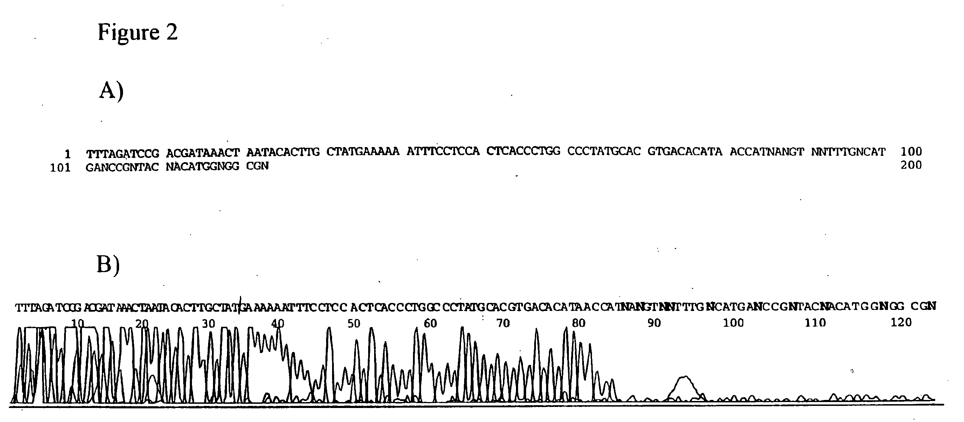

An example of a sequence obtained from a security marker is shown in Figure 2. Figure 2a shows the marker sequence and Figure 2b shows the ABI prism sequence trace.

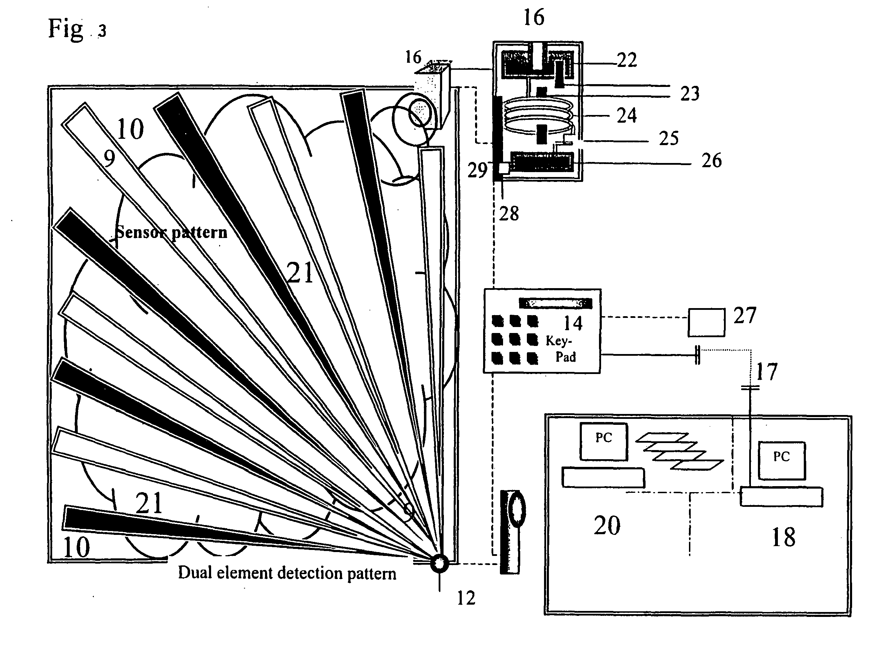

Fig 3 shows an enclosed space such as a large room, this could equally be a trailer, caravan, or any other type of vehicle. Also the area does not necessarily have to be enclosed.

The space indicated at 10 is provided with a conventional movement sensor This can be a dual sensor, either passive infrared and microwave, or passive infrared and ultrasonic to give reliability but in some circumstances conventional single technology will suffice if the area of coverage is not hostile. The detection method is indicated at 12, multiple sensors can be connected to give the correct coverage along with the appropriate warnings and entry exit times. The sensors are connected to a master control panel indicated at 14, the control panel has the facility to store time, date, location, and identify the user with set and unset log etc. It also has the facility to send via GSM or landline indicated at 17 the code of the DNA sequence, with time date and location to a remote monitoring station indicated at 18 on activation therefore automatically up dating the database indicated at 20. The method of communication is via a digital communicator sending a digital code to a digital receiver, the digital receiver has the ability to prioritize and stack calls if required.

On activation the control panel in accordance with preset alarm indication criteria will activate the dispersion apparatus, as the combined device as indicated at 16 this apparatus generates a mist combined with the DNA marker sequence as indicated at 21.

The apparatus 16 generates a mist which fills the space 10 at a rate of 17 m³ per second 340 m³ in 20 seconds, working at + 4 bar (58 PSI). The mist is produced from a heated liquid (134° C) Such as a glycol-based liquid (Ethanol) (CAS NO 64- 17-5) this is held at a precise temperature by two heating elements indicated at 23, one connected to the liquid reservoir indicated at 22 the other to the heat exchanger indicated at 24 to deliver a suitable mist. In the present embodiment the security marker may be introduced in a capillary action, or added directly via the dual valve indicated at 25 or on its own from its own pressurized container indicated at 26. The apparatus 16 has an integral microprocessor indicated at 28 to control its inputs and outputs and operating temperature.

The security marker can also be added to sterile water, CO₂ etc. and pressurized. The controlled release can be via valve, solenoid, explosive cap, pump, to produce a controlled finely divided liquid, jet of vapor or spray.

In the event of an alarm situation occurring, the apparatus 16 is activated filling the area 10 quickly with the solution. At the same time registering the activation along with details of time date location, and unique code to RAM 29 and then sent via the control panel 14 to the control room 18 to be registered on the database 20.

A typical example using a water jet, which could be used in riot and crowd control, to identify people, or objects were present at a given time and place.

The mist as generated by the apparatus 16, will settle on the clothing and exposed parts of the body of the intruders and also on goods within the space 10. The mist will dry to stop cross contamination.

If an intruder is apprehended at a location, or if a person subsequently comes under suspicion or is known by the authorities they can be scanned under controlled conditions and if the covert dye is detected a sample can be taken from the suspect or clothing in the usual way. The same can be done for goods, which are suspected of having been stolen at a location. Using normal forensic science techniques to amplify the security marker DNA by PCR, then by sequencing in two directions to produce data that is accurate and reliable, this is important, as this process will be used in the courts to secure a successful prosecution.

By entering this data on to the secure database to search and retrieve the original matching sequence taken from the original material supplied.

In this way, it is possible to provide proof that a given person or a given object was present at a specified location and at specified time and date.

Another embodiment of the present invention is to use the unique coded solution to impregnate a material for use as a method of authenticity and trace ability of any material or object.

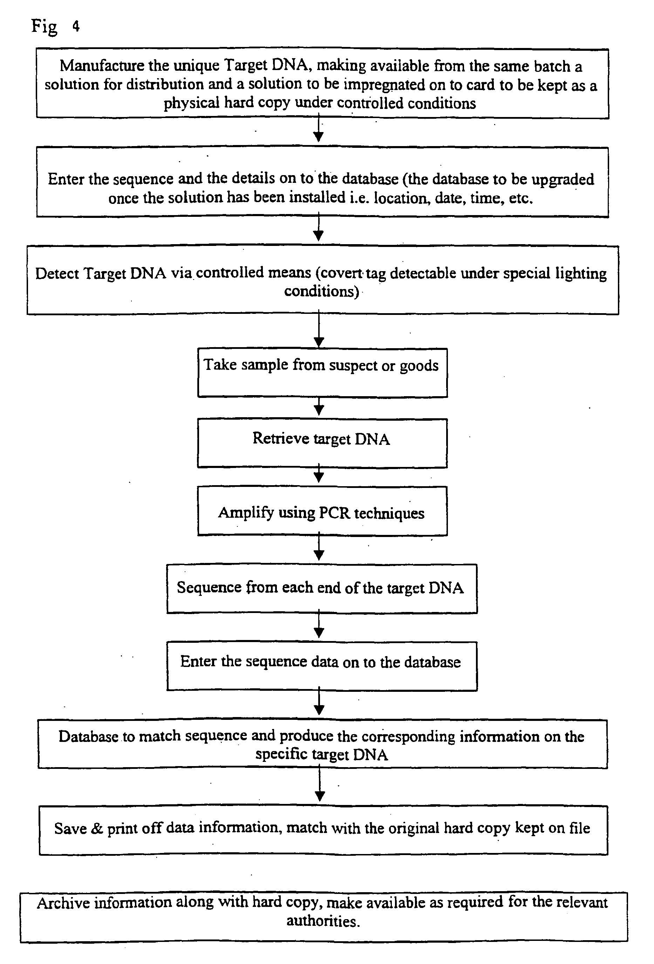

Figure 4 summarises the security marker detection procedure.

In this way, it is possible to provide proof that a given person or a given object was present at a specified location and at a specified time and date.

## Claims

1. A method of producing a security marker for marking objects and/or people, said method comprising:
(A) providing a nucleic acid molecule having a sequence of 40-230 nucleotides, said sequence comprising
(i) a first primer region consisting of 15-50 nucleotides, wherein said first primer region is substantially identical to a first primer, and linked to the first primer region,
(ii) a marker region comprising a predetermined nucleic acid sequence capable of identifying the source of the security marker; and
(iii) a second primer region consisting of 15-50 nucleotides which is substantially the reverse complement of a second primer and wherein said second primer region is capable of being bound by the second primer; and
(B) screening the nucleic acid sequence of the first primer region (or the first primer) and the nucleic acid sequence of the second primer region (or the second primer) against a publicly available database to confirm that the nucleic acid sequences of the first and second primer regions do not occur within 150 bases of each other in a native DNA sequence from humans, dog, cat, mouse, rat, insects or prokaryotic organisms.

2. A method of producing a security marker according to Claim 1, wherein the complementary primer region is downstream of the marker region.

3. A method of producing a security marker according to any preceding claim, wherein the first primer region is different from the second primer region.

4. A method of producing a security marker according to any preceding claim, wherein the or each primer region is derived from the Brown Throated Sloth *(Bradypus variegatus).*

5. A method of producing a security marker according to Claim 4, wherein the or each primer region is derived from the mitochondrial ND1 gene.

6. A method of producing a security marker according to any preceding claim, wherein the marker region consists of between 10 and 30 nucleotides.

7. A method of producing a security marker according to any preceding claim, wherein the or each primer region consists of between 17 and 30 nucleotides.

8. A method of producing a security marker according to any preceding claim, wherein the or each primer region is separated from the marker region by a stretch of 20 to 50 nucleotides.

9. A method of producing a security marker according to any preceding claim, wherein the nucleic acid molecule is labelled.

10. A method of producing a security marker according to Claim 9, wherein the label is a fluorescent dye.

11. A method of producing a security marker according to any preceding claim additionally comprising a separate colorimetric and/or fluorescent label.

12. A method of producing a security marker according to any preceding claim, wherein the nucleic acid molecule comprises at one or both ends a blocking agent.

13. A method of producing a security marker according to Claim 12, wherein the blocking agent comprises a phosphothioate compound, a non-natural nucleoside, a dendromer or an amino alkyl group.

14. A method of producing a security marker according to any preceding claim, wherein the nucleic acid molecule additionally comprises one or more binding agents attached to the nucleic acid molecule.

15. A method of producing a security marker according Claim 14, wherein the binding agent is biotin, vitamin E, cholesterol or a phosphothioate.

16. A method of producing a security marker and attaching said marker to an object and/or person, said method comprising:
(i) producing said security marker by a method according to any of Claims 1-15 and
(ii) applying said security marker to an object.

17. A method according to Claim 16, wherein the security marker is applied by means of a brush, a spray, an aerosol or a stream.

18. A method according to Claim 16 or 17, wherein the object is a human.

19. A method of producing and attaching a security marker to an object and/or person followed by detection of said security marker, said method comprising:
(i) producing and attaching said security marker by a method according to any of Claims 16-18; and
(ii) detecting said security marker by:
(a) obtaining a sample from an object which has been labelled with said security marker;
(b) amplifying the security marker nucleic acid sequence with two primers capable of selectively binding to the marker nucleic acid, wherein in use the first primer binds to a sequence complementary to the first primer region and the second primer binds to the second primer region; and (c) identifying amplified fragments containing the security marker region by size.

20. A method according to Claim 19, wherein the detecting step (ii) further comprises sequencing any security marker identified from at least one primer to determine the nucleic acid sequence of the marker region within the security marker, wherein the security marker identified by the method is preferably amplified prior to sequencing the marker region.

21. A method according to Claim 19 or Claim 20, wherein the security marker is additionally screened with a probe having a region complementary to the marker region.

22. A method according to Claim 20, wherein said sequencing is by dideoxy sequencing, or mass spectrometry sequencing.

23. A method according to any one of Claims 19 to 22, wherein the sequence of the marker region is compared with a database to determine the source of the security marker.

## Patentansprüche

1. Verfahren zum Erzeugen eines Sicherheitsmarkers zum Markieren von Objekten und/oder Personen, wobei das Verfahren die folgenden Schritte beinhaltet:
A) Bereitstellen eines Nukleinsäuremoleküls mit einer Sequenz von 40-230 Nucleotiden, wobei die genannte Sequenz Folgendes umfasst:
i) eine erste Primerregion bestehend aus 15-50 Nucleotiden, wobei die genannte erste Primerregion mit einem ersten Primer im Wesentlichen identisch und mit der ersten Primerregion verbunden ist,
ii) eine Markerregion, die eine vorbestimmte Nucleinsäuresequenz umfasst, die die Quelle des Sicherheitsmarkers identifizieren kann; und
iii) eine zweite Primerregion bestehend aus 15-50 Nucleotiden, die im Wesentlichen das umgekehrte Komplement eines zweiten Primers ist, und wobei die genannte zweite Primerregion von dem zweiten Primer gebunden werden kann; und
B) Screenen der Nucleinsäuresequenz der ersten Primerregion (oder des ersten Primers) und der Nucleinsäuresequenz der zweiten Primerregion (oder des zweiten Primers) anhand einer öffentlich verfügbaren Datenbank, um bestätigen, dass die Nucleinsäuresequenzen der ersten und der zweiten Primerregion nicht innerhalb von 150 Basen voneinander in einer nativen DNA-Sequenz von Menschen, Hunden, Katzen, Mäusen, Ratten, Insekten oder prokaryotischen Organismen auftreten.

2. Verfahren zum Erzeugen eines Sicherheitsmarkers nach Anspruch 1, wobei sich die komplementäre Primerregion stromabwärts von der Markerregion befindet.

3. Verfahren zum Erzeugen eines Sicherheitsmarkers nach einem der vorherigen Ansprüche, wobei sich die erste Primerregion von der zweiten Primerregion unterscheidet.

4. Verfahren zum Erzeugen eines Sicherheitsmarkers nach einem der vorherigen Ansprüche, wobei die oder jede Primerregion vom Braunkehl-Faultier (*Bradypus variegatus)* abgeleitet ist.

5. Verfahren zum Erzeugen eines Sicherheitsmarkers nach Anspruch 4, wobei die oder jede Primer-Region vom mitochondrialen ND1-Gen abgeleitet ist.

6. Verfahren zum Erzeugen eines Sicherheitsmarkers nach einem der vorherigen Ansprüche, wobei die Markerregion aus 10 bis 30 Nucleotiden besteht.

7. Verfahren zum Erzeugen eines Sicherheitsmarkers nach einem der vorherigen Ansprüche, wobei die oder jede Primerregion aus 17 bis 30 Nucleotiden besteht.

8. Verfahren zum Erzeugen eines Sicherheitsmarkers nach einem der vorherigen Ansprüche, wobei die oder jede Primerregion von der Markerregion durch einen Abschnitt von 20 bis 50 Nucleotiden getrennt ist.

9. Verfahren zum Erzeugen eines Sicherheitsmarkers nach einem der vorherigen Ansprüche, wobei das Nucleinsäuremolekül etikettiert ist.

10. Verfahren zum Erzeugen eines Sicherheitsmarkers nach Anspruch 9, wobei das Etikett ein fluoreszierender Farbstoff ist.

11. Verfahren zum Erzeugen eines Sicherheitsmarkers nach einem der vorherigen Ansprüche, der zusätzlich ein separates kolorimetrisches und/oder fluoreszierendes Etikett umfasst.

12. Verfahren zum Erzeugen eines Sicherheitsmarkers nach einem der vorherigen Ansprüche, wobei das Nucleinsäuremolekül einen Blocker an einem oder an beiden Enden umfasst.

13. Verfahren zum Erzeugen einer Sicherheitsmarkers nach Anspruch 12, wobei der Blocker eine Phosphothioatverbindung, ein nichtnatürliches Nucleosid, ein Dendromer oder eine Aminoalkylgruppe umfasst.

14. Verfahren zum Erzeugen eines Sicherheitsmarkers nach einem der vorherigen Ansprüche, wobei das Nucleinsäuremolekül zusätzlich ein oder mehrere Bindemittel umfasst, die an das Nucleinsäuremolekül angelagert sind.

15. Verfahren zum Erzeugen eines Sicherheitsmarkers nach Anspruch 14, wobei das Bindemittel Biotin, Vitamin E, Cholesterol oder ein Phosphthiotat ist.

16. Verfahren zum Erzeugen eines Sicherheitsmarkers und zum Anbringen des genannten Markers an einem Objekt und/oder an einer Person, wobei das genannte Verfahren die folgenden Schritte beinhaltet:
i) Erzeugen des genannten Sicherheitsmarkers mit einem Verfahren nach einem der Ansprüche 1 bis 15 und
ii) Anbringen des genannten Sicherheitsmarkers an einem Objekt.

17. Verfahren nach Anspruch 16, wobei der Sicherheitsmarker mit einem Pinsel, einem Spray, einem Aerosol oder einem Strom angebracht wird.

18. Verfahren nach Anspruch 16 oder 17, wobei das Objekt ein Mensch ist.

19. Verfahren zum Erzeugen und Anbringen eines Sicherheitsmarkers an einem Objekt und/oder einer Person, dann Detektieren des genannten Sicherheitsmarkers, wobei das genannte Verfahren die folgenden Schritte beinhaltet:
i) Erzeugen und Anbringen des genannten Sicherheitsmarkers mit einem Verfahren nach einem der Ansprüche 16-18; und
ii) Detektieren des genannten Sicherheitsmarkers durch:
a) Gewinnen einer Probe von einem Objekt, das mit dem genannten Sicherheitsmarker etikettiert ist;
b) Verstärken der Nucleinsäuresequenz des Sicherheitsmarkers mit zwei Primern, die sich selektiv an die Markernucleinsäure binden können, wobei der erste Primer sich beim Gebrauch an eine Sequenz komplementär zur ersten Primerregion bindet und der zweite Primer sich an die zweite Primerregion bindet; und
c) Identifizieren verstärkter Fragmente, die die Sicherheitsmarker-Region enthalten, anhand der Größe.

20. Verfahren nach Anspruch 19, wobei der Detektierschritt (ii) ferner das Sequenzieren von Sicherheitsmarker beinhaltet, der von wenigstens einem Primer identifiziert wurde, um die Nucleinsäuresequenz der Markerregion innerhalb des Sicherheitsmarkers zu bestimmen, wobei der mit dem Verfahren identifizierte Sicherheitsmarker vorzugsweise vor dem Sequenzieren der Markerregion verstärkt wird.

21. Verfahren nach Anspruch 19 oder Anspruch 20, wobei der Sicherheitsmarker zusätzlich mit einer Sonde gescreent wird, die eine zur Markerregion komplementäre Region hat.

22. Verfahren nach Anspruch 20, wobei die genannte Sequenzierung durch Dideoxy-Sequenzierung oder durch Massenspektrometrie-Sequenzierung erfolgt.

23. Verfahren nach einem der Ansprüche 19 bis 22, wobei die Sequenz der Markerregion mit einer Datenbank verglichen wird, um die Quelle des Sicherheitsmarkers zu bestimmen.

## Revendications

1. Procédé de production d'un marqueur de sécurité pour marquer des objets et/ou des personnes, ledit procédé comprenant :
(A) la mise à disposition d'une molécule d'acide nucléique ayant une séquence de 40 à 230 nucléotides, ladite séquence comprenant
(i) une première région d'amorce constituée de 15 à 50 nucléotides, ladite première région d'amorce étant substantiellement identique à une première amorce, et liée à la première région d'amorce,
(ii) une région marqueur comprenant une séquence d'acide nucléique prédéterminée capable d'identifier la source du marqueur de sécurité ; et
(iii) une deuxième région d'amorce constituée de 15 à 50 nucléotides qui est substantiellement le complément inverse d'une deuxième amorce et ladite deuxième région d'amorce pouvant être liée à la deuxième amorce ; et
(B) le criblage de la séquence d'acide nucléique de la première région d'amorce (ou de la première amorce) et de la séquence d'acide nucléique de la deuxième région d'amorce (ou de la deuxième amorce) contre une base de données disponible dans le commerce pour confirmer que les séquences d'acide nucléique des première et deuxième régions d'amorce ne sont pas présentes dans 150 bases de la région respective, dans une séquence d'ADN native d'être humain, de chien, de chat, de souris, de rat, d'insectes ou d'organismes procaryotes.

2. Procédé de production d'un marqueur de sécurité selon la revendication 1, dans lequel la région d'amorce complémentaire est en aval de la région marqueur.

3. Procédé de production d'un marqueur de sécurité selon l'une quelconque des revendications précédentes, dans lequel la première région d'amorce est différente de la deuxième région d'amorce.

4. Procédé de production d'un marqueur de sécurité selon l'une quelconque des revendications précédentes, dans lequel la ou chaque région d'amorce est dérivée du paresseux à gorge brune *(Bradypus variegatus).*

5. Procédé de production d'un marqueur de sécurité selon la revendication 4, la ou chaque région d'amorce étant dérivée du gène ND1 mitochondrial.

6. Procédé de production d'un marqueur de sécurité selon l'une quelconque des revendications précédentes, dans lequel la région marqueur comprend entre 10 et 30 nucléotides.

7. Procédé de production d'un marqueur de sécurité selon l'une quelconque des revendications précédentes, dans lequel la ou chaque région d'amorce comprend entre 17 et 30 nucléotides.

8. Procédé de production d'un marqueur de sécurité selon l'une quelconque des revendications précédentes, dans lequel le ou chaque région d'amorce est séparée de la région marqueur par un segment de 20 à 50 nucléotides.

9. Procédé de production d'un marqueur de sécurité selon l'une quelconque des revendications précédentes, dans lequel la molécule d'acide nucléique est marquée.

10. Procédé de production d'un marqueur de sécurité selon la revendication 9, dans lequel le marqueur est un colorant fluorescent.

11. Procédé de production d'un marqueur de sécurité selon l'une quelconque des revendications précédentes, comprenant de plus un marqueur colorimétrique et/ou fluorescent séparé.

12. Procédé de production d'un marqueur de sécurité selon l'une quelconque des revendications précédentes, dans lequel la molécule d'acide nucléique comprend à l'une ou aux deux extrémités, un agent bloquant.

13. Procédé de production d'un marqueur de sécurité selon la revendication 12, dans lequel l'agent bloquant comprend un composé phosphothioate, un nucléoside non naturel, un dendromère ou un groupe amino-alkyle.

14. Procédé de production d'un marqueur de sécurité selon l'une quelconque des revendications précédentes, dans lequel la molécule d'acide nucléique comprend en outre un ou plusieurs agents liants fixés à la molécule d'acide nucléique.

15. Procédé de production d'un marqueur de sécurité selon la revendication 14, dans lequel l'agent liant est la biotine, la vitamine E, le cholestérol ou un phosphothioate.

16. Procédé de production d'un marqueur de sécurité et de fixation dudit marqueur à un objet et/ou une personne, ledit procédé comprenant :
(i) la production dudit marqueur de sécurité par un procédé selon l'une quelconque des revendications 1 à 15 et
(ii) l'application dudit marqueur de sécurité à un objet.

17. Procédé selon la revendication 16, dans lequel le marqueur de sécurité est appliqué au moyen d'une brosse, d'un spray, d'un aérosol ou d'un courant.

18. Procédé selon la revendication 16 ou 17, dans lequel l'objet est un être humain.

19. Procédé de production et de fixation d'un marqueur de sécurité à un objet et/ou une personne, suivi de la détection dudit marqueur de sécurité, ledit procédé comprenant :
(i) la production et la fixation dudit marqueur de sécurité par un procédé selon l'une quelconque des revendications 16 à 18 ; et
(ii) la détection dudit marqueur de sécurité par :
(a) l'obtention d'un échantillon issu d'un objet qui a été marqué avec ledit marqueur de sécurité ;
(b) l'amplification de la séquence d'acide nucléique du marqueur de sécurité avec deux amorces capables de se lier sélectivement à l'acide nucléique du marqueur, la première amorce se liant lors de l'utilisation, à une séquence complémentaire à la première région d'amorce et la deuxième amorce se liant à la deuxième région d'amorce ; et
(c) l'identification de fragments amplifiés contenant la région de marqueur de sécurité par taille.

20. Procédé selon la revendication 19, dans lequel l'étape de détection (ii) comprend en outre le séquençage d'un marqueur de sécurité identifié à partir d'au moins une amorce pour déterminer la séquence d'acide nucléique de la région marqueur dans le marqueur de sécurité, le marqueur de sécurité identifié par le procédé étant de préférence amplifié avant le séquençage de la région marqueur.

21. Procédé selon la revendication 19 ou la revendication 20, dans lequel le marqueur de sécurité est en outre criblé avec une sonde ayant une région complémentaire à la région marqueur.

22. Procédé selon la revendication 20, dans lequel ledit séquençage est un séquençage didésoxy ou un séquençage par spectrométrie de masse.

23. Procédé selon l'une quelconque des revendications 19 à 22, dans lequel la séquence de la région marqueur est comparée à une base de données pour déterminer la source du marqueur de sécurité.
